# EUROPEAN PATENT SPECIFICATION

(11) **EP 3 131 866 B1**
(45) Date of publication and mention of the grant of the patent: **27.07.2022**
(21) Application number: 15780476.6
(22) Date of filing: 15.04.2015
(51) Int. Cl.: C05B 17/00, C05C 3/00, C05F 11/00, C05F 11/08, C09K 17/00, C12N 1/20

(54) **NEW SOIL ACTIVATOR CONTAINING AMMONIUM LIGNOSULFONATE, AND USES THEREOF**
NEUER BODENAKTIVATOR MIT AMMONIUM-LIGNINSULFONAT UND VERWENDUNGEN DAVON
NOUVEL ACTIVATEUR DE SOL CONTENANT DU LIGNOSULFONATE D'AMMONIUM, ET SES UTILISATIONS

(30) Priority: 15.04.2014 US 201461979615 P
(43) Date of publication of application: 22.02.2017
(73) Proprietor: Earth Alive Clean Technologies Inc., Montreal, Quebec H4C 2Z6 (CA)
(72) Inventor: GILMOUR, David, Westmount, Québec H3Z 2L7 (CA); YARGEAU, Viviane, St-Basile-le-Grand, Québec J3N 1V3 (CA)
(74) Representative: Regimbeau
(86) International application number: PCT/CA2015/050313
(87) International publication number: WO 2015/157865

(56) References cited:
- WO-A1-92/03393
- WO-A1-96/06531
- WO-A1-2013/096369
- WO-A2-2010/104794
- CA-A1- 2 323 484
- KR-A- 20140 028 779
- US-A- 5 696 094
- US-B1- 6 833 359

## Description

### CROSS REFERENCE TO RELATED APPLICATION

### TECHNICAL FIELD

The present description relates to a soil activator comprising lignosulfonate and a microorganism.

### BACKGROUND ART

Management of soil fertility is a constant pre-occupation of farmers. Plants that are grown in soil require moisture and a number of plant nutrients, such as compounds of nitrogen, phosphorus, potassium, calcium, magnesium, iron, and other essential elements, for vigorous growth. The plant nutrients are usually water soluble and must be in a chemical form that allows them to be utilized by the plants. Thus, the mere presence of essential elemental substances in the soil does not necessarily mean that the plants are able to utilize them effectively as plant nutrients. Also, the plant nutrients should be present in the proper concentrations and ratios for the most effective utilization by the plants. As is well known, many soils are deficient in one or more plant nutrients and/or the plant nutrients that are present are not in a chemical form easily utilized by the plants.

Numerous attempts have been made to overcome the limitations and disadvantages, listed above, of natural soils. The most common approach involves analyzing the soil to determine the available plant nutrients, and then adding the deficient plant nutrients in the form of chemical fertilizers.

Fertilizers are generally an organic or inorganic material that is added to the soil, or in some cases applied directly to foliage, to supply elements essential for the growth and nutrition of plants. Typically, fertilizers provide nitrogen, phosphorus, calcium, magnesium, sulfur and potassium. With increasing knowledge of the role of each nutrients essential to plants there is a better understanding of the importance of providing a given nutrient at the appropriate stage of phenology.

The patent US 5,696,094 discloses a method for controlling soil borne pests and pathogens in which a composition possibly comprising lignosulfonate, agar as nutrient and a blend of microorganisms, is incorporated in the soil.

KR 2014/0028779 describes an antifungal composition for use against soil pathogens in the cultivation of crops, for example with a composition comprising lignosulfonate and a bacteria such as Bacillus subtilis.

US 6,833,359 discloses a method for reducing bacterial and fungal soil pathogens which comprises applying to a soil a chemically effective amount of lignosulfonate possibly in mixture with micro-organisms.

The use of fertilizers can become very expensive since it must be applied repeatedly each year to achieve the best results as all, or part of, the added plant nutrients are consumed by the growing plants. Also, the use of conventional chemical fertilizers does not increase the resistance of the growing plants to disease or adverse environmental conditions.

There is thus still a need to be provided with improved growth supplement or compositions that will increase plant development when applied.

### SUMMARY

According to a first aspect, the present invention relates to a soil activator for stimulating the growth of a plant or crop, comprising:
a) 83% of ammonium lignosulfonate;
b) 10% of at least one microorganism, and
c) 7% of sodium bicarbonate
said ammonium lignosulfonate being at least one of tetrabutyl ammonium lignosulfonate and phenyltrimethylammonium lignosulfonate.

According to a second aspect, the present invention relates to the use of the soil activator as defined above for stimulating the growth of a plant or crop.

In an embodiment, the at least one microorganism is a nitrogen-fixing bacteria, a bacteria releasing nitrogen by degrading organic material or a phosphorus solubilizing bacteria.

In another embodiment, the nitrogen-fixing bacteria is a free-living non-symbiotic bacteria or a mutualistic symbiotic bacteria.

In a further embodiment, the free-living non-symbiotic bacteria is a cyanobacteria, a Azotobacter bacteria, a Bacillus bacteria, a Beijerinckia bacteria, a Klebsiella bacteria, or a Clostridium bacteria.

In another embodiment, the mutualistic symbiotic bacteria is a Rhizobium bacteria or a Azospirillum bacteria.

In an additional embodiment, the phosphorus solubilizing bacteria is a Pantoea bacteria, a Microbacterium laevaniformans bactria or a Pseudomonas bacteria.

In another embodiment, the at least one microorganism is at least one of *Bacillus subtilis, Bacillus licheniformis, Bacillus amyloliquefaciens, Pseudomonas monteilii* and *Pseudomonas putida.*

In another example, the soil activator described herein further comprises at least one of a nutrient.

In another example, the nutrient is at least one of nitrogen, phosphorus, calcium, magnesium, sulfur and potassium.

In another example, the soil activator comprises in dry weight 4.08% of nitrogen, 0.25% of phosphorus (P₂O₅), 0.26% of potassium (K₂O), 6.38% of sulphur, 0.14% of calcium and 0.06% of magnesium.

It is further provided the soil activator described herein for stimulating the growth of a plant or crop.

In an embodiment, the soil activator strengthen the structure and development of the root system and shoot of the plant or crop root system.

In another embodiment, the plant or crop is for arugula, lettuce, radish, spinach, carrots, broccoli, cabbage, cauliflower, cucumbers, onion, garlic, tomatoes, fruit trees, strawberries, corn, wheat, cereal, sorgho, canola, tea, potatoes, potting soil, soya, turf or ornamental plants.

In a further embodiment, the soil activator described herein is for stimulating the growth of a tree.

In an embodiment, the tree is a bamboo tree or a redbud tree.

In another embodiment, the soil activator is applied directly on soil or injected in the soil.

In another embodiment, the soil activator is applied through an irrigation system.

In a further embodiment, the irrigation system is a surface irrigation system, or a sprinkler irrigation system.

In a particular embodiment, the soil activator is dried or liquid.

In a further embodiment, the soil activator is mixed with water.

In a further embodiment, 2g to 20g of soil activator per kg of soil is applied to the soil, injected in the soil or spread on the soil.

In accordance with the present description there is also provided a soil activator comprising a lignosulfonate, nitrogen, phosphorus (P₂O₅), soluble potash (K₂O), *Bacillus subtilis, Bacillus amyloliquefaciens;* and *Pseudomonas monteilii.*

### BRIEF DESCRIPTION OF THE DRAWINGS

Reference will now be made to the accompanying drawings.
Fig. 1 illustrates pictures of arugula plants treated with a soil activator according to one embodiment of the present description, representing a scale of arugula vigor achieved with different treatments, wherein vigor was divided in 5 different categories, each of them representing an increasing degree of plant growth (length) and foliage volume.
Fig. 2 illustrates a graphic representation of the chlorophyll content (A) plant length; (B) and number of plants (C) of the arugulas used on the vigor scale, wherein when possible each column was compare to the first one (Vigor 1) using One-way ANOVA and Dunnett post test (* = statistically significant with a 95 % confidence level (P < 0.05); ** = statistically significant with a 99.9 % confidence level (P < 0.001)).

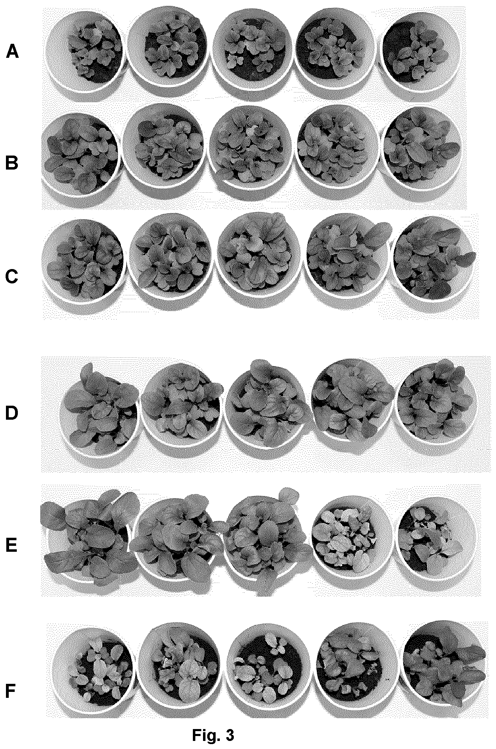

Fig. 3 illustrates the effect of the soil activator described herein on arugula grown on *Pythium* infected soil from the Holland Marsh, pictures of (A) arugula not treated (control), and treated with (B) 2g/Kg; (C) 4g/kg; (D) 8g/kg; (E) 16g/kg and (F) 20 g/kg with the soil activator described herein were taken 2 weeks after planting.
Fig. 4 illustrates the plant vigor after different treatments with increasing concentration of soil activator, wherein each column was compared to the control using One-way ANOVA and Dunnett post test (* = statistically significant with a 95 % confidence level (P < 0.05)).
Fig. 5 illustrates a graphic representation of arugula's dry weight after harvest, wherein each column was compared to the control using One-way ANOVA and Dunnett post test (* = statistically significant with a 95 % confidence level (P < 0.05); ** = statistically significant with a 99.9 % confidence level (P < 0.001)).
Fig. 6 illustrates an arugula's vigor scale, representative scale of arugula vigor achieved with different treatments, wherein vigor was divided in 5 different categories, each of them representing an increasing degree of plant growth (length) and foliage volume on uninfected organic soil.
Fig. 7 illustrates the plant vigor after different treatments with increasing concentration of soil activator in uninfected soil.
Fig. 8 illustrates an arugula's vigor scale, representative scale of arugula vigor achieved with different treatments, wherein vigor was divided in 5 different categories, each of them representing an increasing degree of plant growth (length) and foliage volume on sandy loam soil.
Fig. 9 illustrates the plant vigor after different treatments with increasing concentration of soil activator on sandy loam soil.
Fig. 10 illustrates a tomato's vigour scale, a representative scale of tomato vigor achieved with different treatments, wherein vigor was divided in 5 different categories, each of them representing an increasing degree of plant growth (length) and foliage volume.
Fig. 11 illustrates a graphic representation of the results of tomato plants treated with the soil activator, wherein (A) vigor, (B) number of plants, (C) chlorophyll content, (D) plant length and (E) plant dry weight is measured, when possible each column was compare to the first one (Control) using One-way ANOVA and Dunnett post test (* = statistically significant with a 99.9 % confidence level (P < 0.001); ** = statistically significant with a 99.99 % confidence level (P < 0.0001)).
Fig. 12 illustrates a graphic representation of the vigor of tomato plants after treatment with the soil activator described herein, wherein F indicates presence of fertilizer, and when possible each column was compare to the first one (Control) using One-way ANOVA and Dunnett post test (** = statistically significant with a 99.99 % confidence level (P < 0.0001)).
Fig. 13 illustrates a graphic representation of the (A) number of plants, (B) chlorophyll content, (C) plant length and (D) plant dry weight of tomato plants of Fig. 12, when possible each column was compare to the first one (Control) using One-way ANOVA and Dunnett post test (* = statistically significant with a 99.9 % confidence level (P < 0.001); ** = statistically significant with a 99.99 % confidence level (P < 0.0001)).
Fig. 14 illustrates the average height of eastern redbud treated with the soil activator described herein after 90 days of growth in potting mix, as showed in a picture (A) and histogram (B).
Fig. 15 illustrates the effect on strawberry production of plants treated with the soil activator according to one embodiment.
Fig. 16 illustrates the effect of liquid and powder application of the soil activator according to one embodiment on wheat grown on sandy loam soil, in planter boxes, wherein pictures were taken 15 days after planting.

### DETAILED DESCRIPTION

It is provided a soil activator which act as a naturally powerful biostimulant and soil amendment. The soil activator described herein uses microbial technology in its formulation. The product is a combination of a microorganism and a natural recycled forestry bi-product (lignosulfonates) that provides the conditions microbes need to thrive in the soil, allowing a substantial increase in nutrients uptake by the root systems of plants.

The soil activator described herein contains at least one microorganism. The natural enzymes produced when the soil activator is applied increase nutrients bioavailability in the soil generating a substantial surplus of available micronutrients that the roots take in. This nourishes the plant while boosting the roots strength. By continually enhancing root strength and absorption, the soil activator provided herein helps plants develop stronger, bigger, denser, and healthier root systems for better overall growth and stronger plants that are more resistant to unfavorable environmental conditions.

The microorganism encompassed herein can be for example a nitrogen-fixing bacteria. The microorganisms encompassed herein are thus capable of transforming atmospheric nitrogen into fixed nitrogen, inorganic compounds usable by plants. There are two kinds of nitrogen fixers: free-living (non-symbiotic) bacteria, and mutualistic (symbiotic) bacteria. Free-living (non-symbiotic) bacteria include the cyanobacteria (or blue-green algae). Examples of free-living (non-symbiotic) bacteria are Azotobacter, Bacillus, Beijerinckia, Klebsiella and Clostridium. Mutualistic (symbiotic) bacteria, such as Rhizobium, associated with leguminous plants, and Azospirillum, associated with other plants are also encompassed. Many bacteria also degrade organic matter, releasing fixed nitrogen for reuse by other organisms. Furthermore, the microorganism encompassed herein can also be a bacteria releasing nitrogen by degrading organic material. In addition the microorganism encompassed herein can also be a phosphorus solubilizing bacteria. Phosphorus solubilizing bacteria are also important as they are capable of hydrolyzing organic and inorganic phosphorus from insoluble compounds and includes Pantoea agglomerates strain, Microbacterium laevaniformans strain and Pseudomonas putida, which have been identified as the highly efficient insoluble phosphate solubilizer.

Accordingly, but not limited to, the microorganisms include for example *Bacillus subtilis, Bacillus licheniformis,* and *Pseudomonas putida.*

The disclosed soil activator comprises principally lignosulfonate. For example, the composition described herein comprises up to 83% of lignosulfonate. The composition as claimed comprises 83% of ammonium lignosulfonate. Lignosulfonates, or sulfonated lignin, are water-soluble anionic polyelectrolyte polymers. They are byproducts from the production of wood pulp using sulfite pulping. Lignosulfonates are recovered from the spent pulping liquids (red or brown liquor) from sulfite pulping. The most widely used industrial process is the Howard process, in which 90-95% yields of calcium lignosulfonates, are precipitated by adding of excess calcium hydroxide. Ultrafiltration and ion-exchange can also be used to separate lignosulfonates from the spent pulping liquid.

Preferably, the soil activator described herein comprises ammonium lignosulfonate. An ammonium lignosulfonate salt may be used, e.g., a tetraalkyl ammonium or aryltrialkylammonium counterion may be used. Examples of these types of dispersants include tetrabutyl ammonium lignosulfonate and phenyltrimethylammonium lignosulfonate.

The composition as claimed herein comprises ammonium lignosulfonate (ALS) (83%), microorganisms (10%), and sodium bicarbonate (7%).

In another embodiment, the soil activator provided herein can be mixed and applied with most crop production products and crop protection products including nutrient solutions, fertilizers, insecticides, herbicides and fungicides. The mixing doses will vary according to the cultivation, its development stage, the soil properties and the application modalities.

Accordingly, the soil activator described herein can comprise nutrients such as nitrogen, phosphorus, calcium, magnesium, sulfur and potassium.

The composition can also comprise the nutrients in a quantity as disclosed in Table 1:

**Table 1**

| Soil activator composition | |
|---|---|
| Product composition | (%) Dry weight |
| Total nitrogen | 4.08 |
| Phosphorus (P₂O₅) | 0.25 |
| Potassium (soluble K₂O) | 0.26 |
| Sulphur | 6.38 |
| Calcium | 0.14 |
| Magnesium | 0.06 |

The soil activator provided herein is used to strengthen the structure and development of the root system and shoot of any type of plant. As disclosed herein, the soil activator described herein has a positive impact on arugula growth. As demonstrated, the soil activator has a positive impact in arugula growth independent of the soil where it is planted.

The soil activator also shows a noticeable positive effect on tomato plants growth, independent of the use of fertilizer.

The use of the soil activator also shows positive effect on eastern redbud (*Cerci canadensis*)*.*

The disclosed composition is perfectly suited for a wide range of applications such as conventional farming (small and large scale), orchards, shrubs and evergreens, hothouses and hydroponics, large-scale flower growing and home vegetable. As shown herein, the use of the soil activator increased the biomass produced of carrots and onions in field testing.

The soil activator provided herein is used to strengthen the structure and development of the root system and shoot of a plant or crop such as for example, arugula, lettuce, radish, spinach, broccoli, cabbage, cauliflower, cucumbers, carrots, onion, garlic, tomatoes, fruit trees, strawberries, corn, wheat, cereal, sorgho, canola, tea, potatoes, potting soil, turf and/or ornamental plants.

In an embodiment, the soil activator may be soil applied or injected through irrigation systems such as surface irrigation, sprinkler irrigation including center pivot and through drip irrigation. For sprinkler irrigation, the soil activator is preferably mixed with water before introducing to the sprinkler system.

The present invention will be more readily understood by referring to the following examples, which are given to illustrate embodiments rather than to limit its scope.

### EXAMPLE I

### Growth of treated arugula crops

Microgreens and petite greens are young edible greens produced from various kinds of vegetables, herbs or other plants. They are harvested before they develop into larger plants and despite their small size they have intense flavor and color. There is an increasing demand for these products from upscale markets and fine dining restaurants.

Arugula, is an example of a fast grower widely consumed petite green.

Arugula seeds were used and different soils mixed with different concentration of the soil activator comprising ammonium lignosulfonate (ALS) (83%), microorganisms (10%; *Bacillus subtilis, Bacillus licheniformis,* and *Pseudomonas putida*), and sodium bicarbonate (7%). 360 g of soil (50 % humidity for sandy loam soil and 80 % humidity for the Holland Marsh soil) were mixed with different concentration of dry soil activator and kept in a seal plastic bag for 48 h. Ten arugula seeds were planted on 60 g of the treated soil per pot and five pots were used per treatment (n=5). Plant emergence and growth were monitored and after two weeks plant were harvested, processed and the results analyzed. Each experiment was repeated twice.

The experiments were repeated on different soils, including a *Phytium-infected* soil. Specifically, Arugula seeds were planted in sandy loam soil, organic soil from the Holland Marsh and Holland Marsh soil infected with *Pythium.* Results demonstrated that the soil activator has a positive impact in arugula growth independent of the soil were it is planted.

The selected treatments were:
- Control (n=5)
- soil activator 2 g/kg of soil (n=5)
- soil activator 4 g/kg of soil (n=5)
- soil activator 8 g/kg of soil (n=5)
- soil activator 16 g/kg of soil (n=5); and
- soil activator 20 g/kg of soil (n=5).

360 g of soil (50 % humidity for sandy loam soil and 80 % humidity for the Holland Marsh soil) were mixed with different concentration of dry soil activator and kept in a seal plastic bag for 48 h. Ten arugula seeds were planted on 60 g of the treated soil per pot and five pots were used per treatment (n=5). Plant emergence and growth were monitored and after two weeks, plants were harvested, processed and the results analyzed. Each experiment was repeated twice.

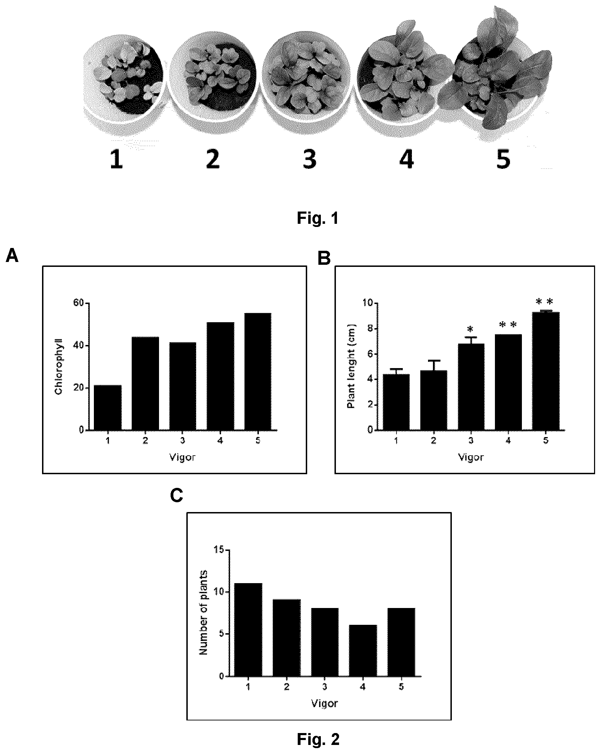

For about 8 days all the plants treated with the soil activator looked stunted as compare with the untreated control. However, after that the soil activator has a noticeable positive effect on plant growth at most of the concentration used. Two weeks after planting, the arugulas were processed, the number of plants was counted and the chlorophyll content, shoot length, total plant length, and total plant dry weight were measured. An arugula's vigor scale as a visual way of measuring plant growth and foliage volume was created (Fig. 1).

While chlorophyll content and length increased with the vigor of the plant, there was no correlation between vigor and number of plants (Fig. 2).

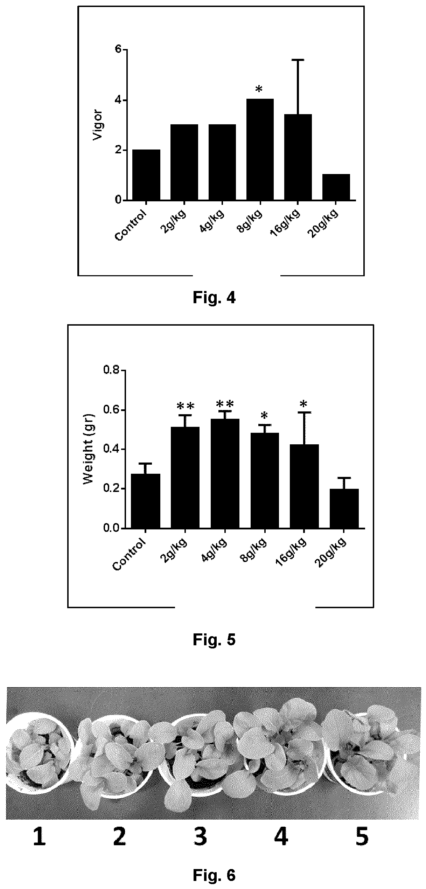

Using the vigor scale described herein, the plants were rated on each pot of each treatment. As shown in Fig. 4, arugula's vigor increased whith increasing concentration of the soil activator, reaching a maximun when 8 g/kg were mixed with the contaminated soil. The use of 20 g/kg of soil activator was toxic, resulting in small yellowish plants. The differences between pots obtained when 16 g/kg of soil activator was added, are most likely due to incomplet or improper mix of the product with the soil. Similar results were obtained on a second set of experiments, confirming that 8 g of soil activator per kg of *Pythium*-infected soil is the optimum concentration to obtain the maximum arugula vigor.

Harvested arugulas were dry overnight at 80 °C and the dry weight was measured. A correlation between increasing concentration of soil activator and increasing dry weight was found, demonstrating that the soil activator described herein increases plant growth and foliage volume (Fig. 5).

The soil activator also had a positive impact on arugulas grown on uninfected organic soil. Considering that is a different soil, a new arugula's vigor scale was created as a visual way of measuring plant growth and foliage volume (Fig. 6).

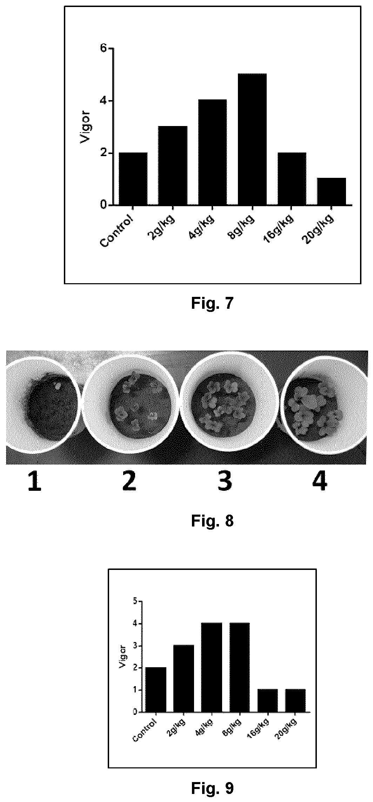

As shown in Fig. 7, by mixing 8 g of soil activator per kilo of soil, the higher plant vigor was obtained.

Arugulas did not grow well in the sandy loam soil. Despite the poor growth, the same positive effect of the soil activator described herein on plants growth was observed. Once again the best result was obtained when 8 g of soil activator per kg of soil was mixed. The soil activator was toxic when used at 16 g or 20 g per kg of soil (Fig. 9). A new arugula's vigor scale was created as a visual way of measuring plant growth and foliage volume (Fig. 8).

### EXAMPLE II

### Growth of treated tomatoes crops

360 g of potting mix (80 % humidity) were mixed with different concentration of dry soil activator comprising ammonium lignosulfonate (ALS) (83%), microorganisms (10%; *Bacillus subtilis, Bacillus licheniformis,* and *Pseudomonas putida*), and kept in a seal plastic bag for 48 h.

Tomato seeds (variety TSH04) and Sunshine potting mix were used, with or without fertilizer (0.5 g/L miracle growth, NPK = 28:8:16) mixed with different concentration of soil activator. The selected treatments were:
- Control (n=5)
- soil activator 2 g/kg of soil (n=10)
- soil activator 4 g/kg of soil (n=10)
- soil activator 8 g/kg of soil (n=10)
- soil activator 16 g/kg of soil (n=10); and
- soil activator 20 g/kg of soil (n=10).

360 g of soil (80 % humidity) were mixed with different concentration of dry and kept in a seal plastic bag for 48 h. 4 tomato seeds were planted on 60 g of the treated soil per pot and five pots were used per treatment (n=10). After germination, 35 ml of miracle growth fertilizer (0.5 g/L) were added to the designated pots. Plant emergence and growth were monitored after 25 days plants were harvested, processed and the results analyzed. Each experiment was repeated twice.

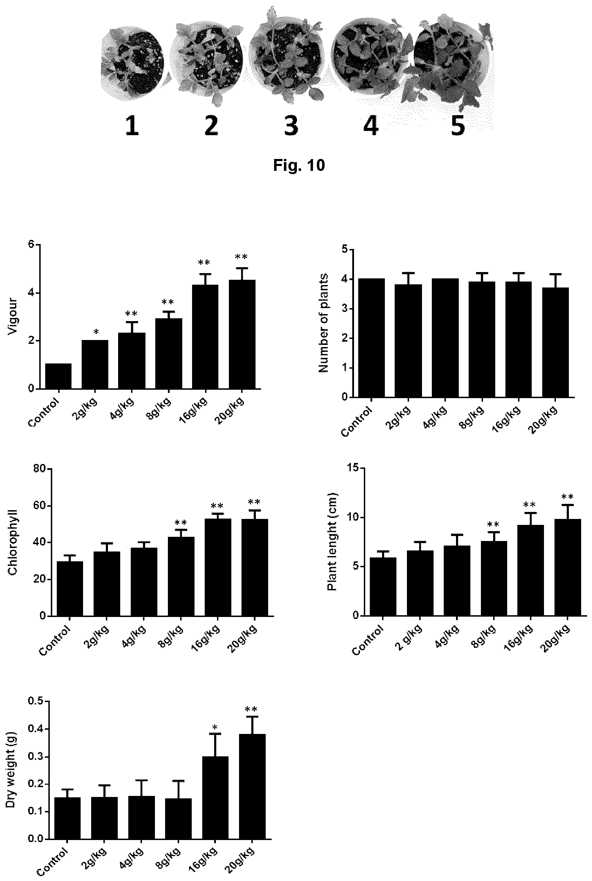

The soil activator described herein has a noticeable positive effect on plant growth at most of the concentration used. 25 days after planting, tomato plants were processed, the number of plants was counted and the chlorophyll content, total plant length, and total plant dry weight were measured. A tomato's vigor scale was created as a visual way of measuring plant growth and foliage volume (Fig. 10).

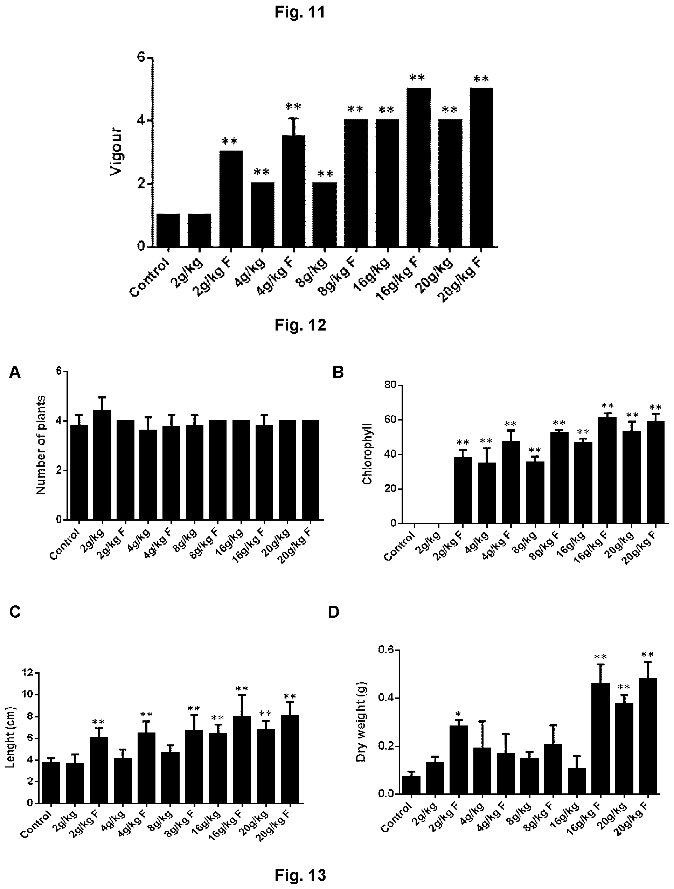

Using the vigour scale described hereinabove, the plants were rated on each pot of each treatment. As shown in Fig. 11A, tomato's vigour increased whith increasing concentration of soil activator used, reaching a maximun when 16 g/kg were mixed with the potting mix. The number of plants was not affected by the soil activator, suggesting that the soil activator does not affect germination rates on tomato plants (Fig. 11B). Chlorophyll content and plant length also increased when at least 8 g of the soil activator per kg of potting mix were used (Figs. 11C and D). Harvested tomato plants were dry overnight at 80°C and the dry weight was measured. A statistically significant increase was found on dry weight when at least 16 g of the soil activator per kg of potting mix were used, demonstrating that the soil activator increases plant growth and foliage volume (Fig. 11E).

In general, increasing concentrations of the soil activator positively impacted all the parameters recorded except for the number of plants. The best results were obtained when 16g and 20g per kg of potting mix were used; suggesting that for tomato, the optimal concentration of the soil activator needed is within that range.

The soil activator describe herein has a positive effect on tomato plant growth when used on fertilized potting mix. To evaluate the growth promotion ability of the soil activator without fertilizer, the previous experiment was repeated in the presence and absence of miracle growth fertilizer. This time the number of samples per treatment was 5 and as control fertilized tomato plants were used.

As shown in Fig. 12, tomato's vigour increased whith increasing concentration of soil activator, reaching a maximun when 16 g/kg were mixed with the potting mix independent of the presence of fertilizer.

The soil activator did not affect tomatoes germination rate (Fig. 13A), but it had a positive effect on chlorophyll content and plant length. These parameters also reached a maximum when 16 g per kg of potting mix were used, independently of the presence of fertilizer (Figs. 13B and C). A significant increase was obtained on plant dry weight when 16 g/kg of soil activator were used on fertilized potting mix or when 20g/g of soil activator were used independently of the presence of fertilizer (Fig. 13D).

### EXAMPLE III

### Growth of treated eastern redbud

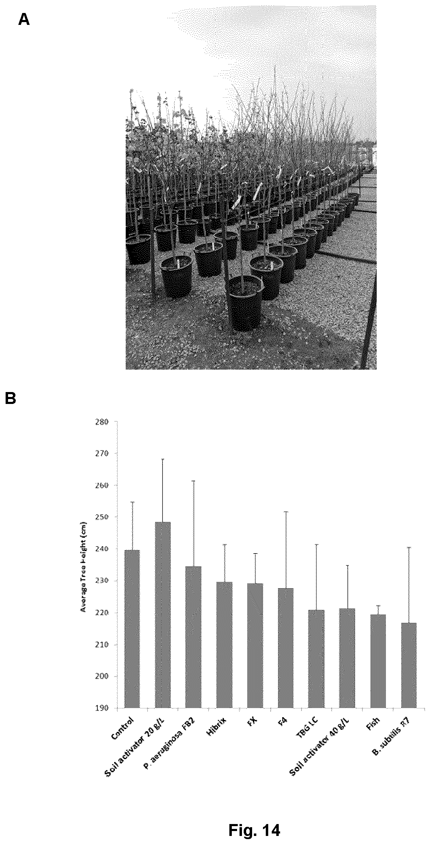

The soil activator (comprising ammonium lignosulfonate (ALS) (83%), microorganisms (10%; *Bacillus subtilis, Bacillus licheniformis,* and *Pseudomonas putida*) was mixed with water at a concentration of 20 g/L and 40 g/L. Treatments were poured directly onto the pots of eastern redbud (500 mL per 10 gallon pots), n = 5 (see Fig. 14B).

The use of the soil activator has a positive effect on trees height when applied at a rate of 20 g/L (see Fig. 14A).

### EXAMPLE IV

### Field testing on carrots and onions growth

Results on mass of vegetable (carrots and onions) produced were obtained. The results summarized in Table 1 below indicate that for both crops, the use of the soil activator increased the biomass produced.

**Table 1**

| Preliminary results based on mass of vegetable produced | | | |
|---|---|---|---|
| Vegetable | kg vegetable harvested/plot (mean ± std) | | |
| | Control | Inorganic fertilizer (IF) | Soil activator |
| Carrots | 8.98 ±0.47 | 11.10 ±0.64 | 14.68 ±1.27 |
| Onions | 4.79 ±1.27 | 4.97 ±1.86 | 6.38 ±1.74 |

### EXAMPLE V

### Growth of treated strawberry plants

The field consisting of a total area of 522,6 m² (5,625 square feet) planted with 12 rows each containing 650 strawberry plants spaced approximately 15 cm (6 inches) apart was used. 200 Albion strawberry seedlings per treatment were planted in the middle of the field on a single row with 50 control plants (pre-treated with water) planted at the end of each row. Nine different treatments were applied in this field trial.

Strawberry seedlings were kept at 4 °C. One day before planting, seedlings were soaked in 6 liters of soil activator 10 g/L. Seedlings were first completely submerged in the treatment solution and then roots were left soaking for 4 hrs. Control plants were treated with water. Two and a half months after planting, plants were re-treated by drenching each plant with approximately 30 ml of the respective treatment.

Strawberry seedlings were planted by hand. Soil was fertilized with 4.5 kilos (10 lbs) per week of equal amounts of calcium nitrate and potassium nitrate. Plant growth was monitored at the sixth and eleventh week after planting by measuring chlorophyll content, leaf height and width.

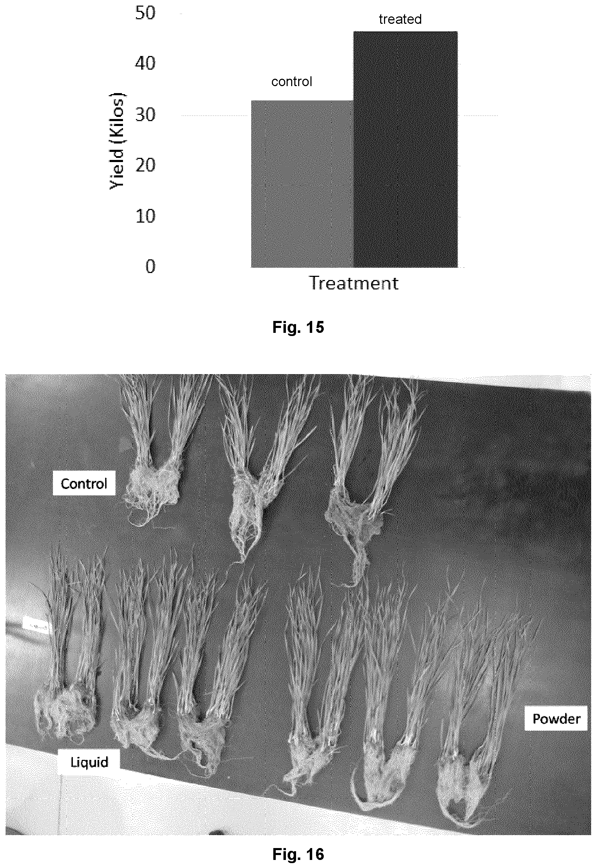

Once harvest season began, fruits were collected every two days. It was observed that treated plants produced higher yields than control plants during the whole season and production stayed more uniform over time. Data collected after the final harvest showed that plants treated with the soil activator produced 46.34 kilos (102.17 lbs) while control plants produced 32.8 kilos (72.23 lbs) (Fig. 15). This represents a 41% increase in yields.

Data were analyzed using the SAS program and the General Linear Model (GLM) Procedure. This procedure gives the results of three different statistical tests including T-Tests, Duncan's Multiple Range Test and Tukey's Studentized Range.

Treating strawberry seedlings with soil activator described herein before planting improved plant growth at early stages and more importantly, increased strawberry production by 41% as compared to control plants.

### EXAMPLE VI

### Effect of liquid or powder application of the soil activator on wheat plants

Wheat seeds in rows were planted in planter boxes to test liquid and powder application of the soil activator. Seeds were planted 30 per row, 2 rows per box, 3 boxes per treatment.

The seeds were planted at the bottom of small trenches in sandy loam soil, and then the sides of the trenches were pushed in to cover the seeds. The powder was sprinkled to cover the bottom of the trench, and seeds were placed on top. For liquid application, 6mL/row of 600g/L solution of the soil activator was poured along the bottom of the trench, and seeds were again placed on top.

A noticeable positive effect was observed from both powder and liquid application of the soil activator on wheat growth (Fig 16). Particularly for the liquid application, a significant increase on chlorophyll content, plant length and shoot dry weight was observed. Notably, the use of the soil activator as a liquid at 6g/Kg soil showed the most improvement on the wheat growth.

Similarly, treatments with 3 g or 6 g of the soil activator (powder) per Kilo of soil improve significantly wheat growth.

While the invention has been described in connection with specific embodiments thereof, it will be understood that it is capable of further modifications and this application is intended to cover any variations, uses, or adaptations of the invention, including such departures from the present disclosure as come within known or customary practice within the art to which the invention pertains and as may be applied to the essential features hereinbefore set forth, and as follows in the scope of the appended claims.

## Claims

1. A soil activator for stimulating the growth of a plant or crop, comprising:
a) 83% of ammonium lignosulfonate;
b) 10% of at least one microorganism, and
c) 7% of sodium bicarbonate
said ammonium lignosulfonate being at least one of tetrabutyl ammonium lignosulfonate and phenyltrimethylammonium lignosulfonate.

2. The soil activator of claim 1, wherein said at least one microorganism is a nitrogen-fixing bacteria, a bacteria releasing nitrogen by degrading organic material or a phosphorus solubilizing bacteria.

3. The soil activator of claim 2, wherein said nitrogen-fixing bacteria is a free-living non-symbiotic bacteria or a mutualistic symbiotic bacteria.

4. The soil activator of claim 3, wherein the free-living non-symbiotic bacteria is a cyanobacteria, a Azotobacter bacteria, a Bacillus bacteria, a Beijerinckia bacteria, a Klebsiella bacteria, or a Clostridium bacteria.

5. The soil activator of claim 3, wherein said mutualistic symbiotic bacteria is a Rhizobium bacteria or a Azospirillum bacteria.

6. The soil activator of claim 2, wherein said phosphorus solubilizing bacteria is a Pantoea bacteria, a Microbacterium laevaniformans bactria or a Pseudomonas bacteria.

7. The soil activator of claim 1, wherein said at least one microorganism is at least one of *Bacillus subtilis, Bacillus licheniformis, Bacillus amyloliquefaciens, Pseudomonas monteilii* and *Pseudomonas putida.*

8. The soil activator of any one of claims 1 to 7, wherein the soil activator strengthens the structure and development of the root system and shoot of the plant or crop root system.

9. The soil activator of claim 8, wherein the soil activator increases the chlorophyll content of the plant.

10. The soil activator of claim 8 or 9, wherein the plant or crop is arugula, lettuce radish, spinach, carrots, broccoli, cabbage, cauliflower, cucumbers, onion, garlic, tomatoes, fruit trees, strawberries, corn, wheat, cereal, sorgho, canola, tea, potatoes, potting soil, soya, turf or ornamental plants.

11. The soil activator of any one of claims 1-10, for stimulating the growth of a tree.

12. The soil activator of claim 11, wherein said tree is a bamboo tree or a redbud tree.

13. The soil activator of any one of claims 8-12, wherein said soil activator is applied directly on soil or injected in the soil.

14. The soil activator of claim 13, wherein the soil activator is applied through an irrigation system.

15. The soil activator of claim 14, wherein the irrigation system is a surface irrigation system, or a sprinkler irrigation system.

16. The soil activator of any one of claims 1-15, wherein said soil activator is dried or liquid.

17. The soil activator of claim 16, wherein said soil activator is mixed with water.

18. The soil activator of claim 13, wherein 2g to 20g of soil activator per kg of soil is applied to the soil, injected in the soil or spread on the soil.

19. Use of a soil activator as defined in any one of claims 1 to 18 for stimulating the growth of a plant or crop.

## Patentansprüche

1. Bodenaktivator zur Stimulation des Wachstums einer Pflanze oder Kulturpflanze, umfassend:
a) 83 % Ammoniumlignosulfonat;
b) 10 % mindestens eines Mikroorganismus, und
c) 7 % Natriumbicarbonat,
wobei das Ammoniumlignosulfonat mindestens eines von Tetrabutylammoniumlignosulfonat und Phenyltrimethylammoniumlignosulfonat ist.

2. Bodenaktivator nach Anspruch 1, wobei der mindestens eine Mikroorganismus eine stickstoffbindende Bakterie, eine Bakterie, die Stickstoff durch Abbauen von organischem Material freisetzt, oder eine phosphorsolubilisierende Bakterie ist.

3. Bodenaktivator nach Anspruch 2, wobei die stickstoffbindende Bakterie eine frei lebende, nichtsymbiotische Bakterie oder eine wechselseitig symbiotische Bakterie ist.

4. Bodenaktivator nach Anspruch 3, wobei die frei lebende, nichtsymbiotische Bakterie eine Cyanobakterie, eine Azotobacter-Bakterie, eine Bacillus-Bakterie, eine Beijerinckia-Bakterie, eine Klebsiella-Bakterie oder eine Clostridium-Bakterie ist.

5. Bodenaktivator nach Anspruch 3, wobei die wechselseitig symbiotische Bakterie eine Rhizobium-Bakterie oder eine Azospirillum-Bakterie ist.

6. Bodenaktivator nach Anspruch 2, wobei die phosphorsolubilisierende Bakterie eine Pantoea-Bakterie, eine Microbacterium-laevaniformans-Bakterie oder eine Pseudomonas-Bakterie ist.

7. Bodenaktivator nach Anspruch 1, wobei der mindestens eine Mikroorganismus mindestens einer von *Bacillus subtilis, Bacillus licheniformis, Bacillus amyloliquefaciens, Pseudomonas monteilii* und *Pseudomonas putida* ist.

8. Bodenaktivator nach einem der Ansprüche 1 bis 7, wobei der Bodenaktivator die Struktur und die Entwicklung des Wurzelsystems und den Spross des Pflanzen- oder Kulturpflanzenwurzelsystems stärkt.

9. Bodenaktivator nach Anspruch 8, wobei der Bodenaktivator den Chlorophyllgehalt der Pflanze erhöht.

10. Bodenaktivator nach Anspruch 8 oder 9, wobei es sich bei der Pflanze oder Kulturpflanze um Rucola, Kopfsalat, Radieschen, Spinat, Mohrrüben, Brokkoli, Kohl, Blumenkohl, Gurken, Zwiebel, Knoblauch, Tomaten, Obstbäume, Erdbeeren, Mais, Weizen, Getreide, Sorgho, Raps, Tee, Kartoffeln, Topferde, Sojabohne, Rasen oder Zierpflanzen handelt.

11. Bodenaktivator nach einem der Ansprüche 1 bis 10 zur Stimulation des Wachstums eines Baums.

12. Bodenaktivator nach Anspruch 11, wobei der Baum ein Bambusbaum oder ein Judasbaum ist.

13. Bodenaktivator nach einem der Ansprüche 8 bis 12, wobei der Bodenaktivator direkt auf Boden aufgebracht oder in den Boden gespritzt wird.

14. Bodenaktivator nach Anspruch 13, wobei der Bodenaktivator durch ein Bewässerungssystem aufgebracht wird.

15. Bodenaktivator nach Anspruch 14, wobei das Bewässerungssystem ein Oberflächenbewässerungssystem oder ein Bewässerungsregnersystem ist.

16. Bodenaktivator nach einem der Ansprüche 1 bis 15, wobei der Bodenaktivator getrocknet oder flüssig ist.

17. Bodenaktivator nach Anspruch 16, wobei der Bodenaktivator mit Wasser gemischt wird.

18. Bodenaktivator nach Anspruch 13, wobei 2 g bis 20 g Bodenaktivator pro kg Boden auf den Boden aufgebracht, in den Boden gespritzt oder auf dem Boden verteilt wird.

19. Verwendung eines Bodenaktivators nach einem der Ansprüche 1 bis 18 zur Stimulation des Wachstums einer Pflanze oder Kulturpflanze.

## Revendications

1. Activateur de sol pour stimuler la croissance d'une plante ou d'une culture, comprenant :
a) 83 % de lignosulfonate d'ammonium,
b) 10 % d'au moins un microorganisme, et
c) 7 % de bicarbonate de sodium,
ledit lignosulfonate d'ammonium étant au moins l'un parmi le lignosulfonate de tétrabutylammonium et le lignosulfonate de phényltriméthylammonium.

2. Activateur de sol selon la revendication 1, dans lequel ledit au moins un microorganisme est une bactérie fixant l'azote, une bactérie libérant l'azote par dégradation de matière organique, ou une bactérie solubilisant le phosphore.

3. Activateur de sol selon la revendication 2, dans lequel ladite bactérie fixant l'azote est une bactérie non symbiotique libre ou une bactérie symbiotique mutualiste.

4. Activateur de sol selon la revendication 3, dans lequel la bactérie non symbiotique libre est une cyanobactérie, une bactérie Azotobacter, une bactérie Bacillus, une bactérie Beijerinckia, une bactérie Klebsiella, ou une bactérie Clostridium.

5. Activateur de sol selon la revendication 3, dans lequel ladite bactérie symbiotique mutualiste est une bactérie Rhizobium ou une bactérie Azospirillum.

6. Activateur de sol selon la revendication 2, dans lequel ladite bactérie solubilisant le phosphore est une bactérie Pantoea, une bactérie Microbacterium laevaniformans ou une bactérie Pseudomonas.

7. Activateur de sol selon la revendication 1, dans lequel ledit au moins un microorganisme est au moins l'un parmi *Bacillus subtilis, Bacillus licheniformis, Bacillus amyloliquefaciens, Pseudomonas monteilii* et *Pseudomonas putida.*

8. Activateur de sol selon l'une quelconque des revendications 1 à 7, dans lequel l'activateur de sol renforce la structure et le développement du système racinaire et les pousses du système racinaire de la plante ou de la culture.

9. Activateur de sol selon la revendication 8, dans lequel l'activateur de sol augmente la teneur en chlorophylle de la plante.

10. Activateur de sol selon la revendication 8 ou 9, dans lequel la plante ou la culture est la roquette, la laitue, le radis, l'épinard, la carotte, le brocoli, le chou, le chou-fleur, le concombre, l'oignon, l'ail, la tomate, les arbres fruitiers, la fraise, le maïs, le blé, les céréales, le sorgho, le canola, le thé, la pomme de terre, le terreau, le soja, le gazon ou les plantes ornementales.

11. Activateur de sol selon l'une quelconque des revendications 1 à 10, pour stimuler la croissance d'un arbre.

12. Activateur de sol selon la revendication 11, dans lequel ledit arbre est un bambou ou un arbre de Judée.

13. Activateur de sol selon l'une quelconque des revendications 8 à 12, dans lequel ledit activateur de sol est appliqué directement sur le sol ou injecté dans le sol.

14. Activateur de sol selon la revendication 13, dans lequel l'activateur de sol est appliqué par l'intermédiaire d'un système d'irrigation.

15. Activateur de sol selon la revendication 14, dans lequel le système d'irrigation est un système d'irrigation en surface, ou un système d'irrigation par aspersion.

16. Activateur de sol selon l'une quelconque des revendications 1 à 15, dans lequel ledit activateur de sol est séché ou liquide.

17. Activateur de sol selon la revendication 16, dans lequel ledit activateur de sol est mélangé avec de l'eau.

18. Activateur de sol selon la revendication 13, dans lequel 2 g à 20 g d'activateur de sol par kg de sol sont appliqués au sol, injectés dans le sol ou répandus sur le sol.

19. Utilisation d'un activateur de sol tel que défini dans l'une quelconque des revendications 1 à 18 pour stimuler la croissance d'une plante ou d'une culture.
